# EUROPEAN PATENT SPECIFICATION

(11) **EP 3 369 515 B1**
(45) Date of publication and mention of the grant of the patent: **10.11.2021**
(21) Application number: 18159726.1
(22) Date of filing: 02.03.2018
(51) Int. Cl.: B23K 9/16, A61F 9/06, B23K 9/32, G02B 27/01, G10K 11/00

(54) **WELDING SYSTEM WITH A HELMET HAVING HEADS UP DISPLAY AND VOICE COMMAND**
SCHWEISSSYSTEM MIT HELM MIT HEADUP-ANZEIGE UND SPRACHSTEUERUNG
SYSTÊME DE SOUDAGE AVEC UN CASQUE AYANT UN AFFICHAGE ET UNE COMMANDE VOCALE

(30) Priority: 02.03.2017 US 201715447240
(43) Date of publication of application: 05.09.2018
(73) Proprietor: Lincoln Global, Inc., Santa Fe Springs, CA 90670 (US)
(72) Inventor: SUMNER, Steven R., Brunswick, OH Ohio 44212 (US)
(74) Representative: Grosse Schumacher Knauer von Hirschhausen

(56) References cited:
- EP-A1- 1 025 946
- US-A- 6 061 456
- US-A1- 2010 223 706
- US-A1- 2015 209 887
- US-A1- 2016 125 653
- US-A1- 2016 267 806

## Description

This invention relates to a welding system according to the preamble of claim 1 (see for example US 2016/125653 A1), and thus in general to equipment used in welding. Devices, systems, and methods consistent with the invention relate to the monitoring of welding parameters and specifically to a welding helmet with a head up display (HUD) and voice command and control features.

### Description of the Related Art

Welding is an important process in the manufacture and construction of various products and structures. Applications for welding are widespread and used throughout the world, for example, the construction and repair of ships, buildings, bridges, vehicles, and pipe lines, to name a few. Welding may performed in a variety of locations, such as in a factory with a fixed welding operation or on site with a portable welder.

In manual or semi-automated welding a user/operator (i.e. welder) directs welding equipment to make a weld. For example, in arc welding the welder may manually position a welding rod or welding wire and produce a heat generating arc at a weld location. In this type of welding the spacing of the electrode from the weld location is related to the arc produced and to the achievement of optimum melting/fusing of the base and welding rod or wire metals. The quality of such a weld is often directly dependent upon the skill of the welder.

Welders generally rely upon a variety of information when welding. This information includes, for example, current and voltage. Traditionally, welders would need to look at gauges on the control panel of the welding equipment to gain this information. This would require the welder to direct their field of vision away from the welding work area and as such was undesirable. In addition, in many cases, the welding machine may not be located close to the work space. In such cases, the welding machine is operated by a cable-connected remote control that can be used to change parameters such as, e.g., welding power, polarity, arc characteristics, etc. However, before the process can be set up, the welder may need to see the display readouts that are physically located on the machine. The setting-up process may require many trips before the set-up is completed.

In the past, efforts have been made to provide welders with information during welding, such as in the method disclosed in U.S. Pat. No. 4,677,277, where current and voltage are monitored to produce an audio indication to the operator as to the condition of the arc in arc welding. However, monitors consisting only of audio arc parameter indicators are hard to hear and interpolate and are not capable of achieving the desired closeness of control and quality of weld often required. More recently, as disclosed in U.S. Pat. No. 6,242,711, an apparatus for monitoring arc welding has been developed that provides a welder with real-time voltage and current conditions of the welding arc where information in the form of lights, illuminated bar graphs, light projections, illuminated see-through displays, or the like are placed within the visual range of the helmet wearing operator and located in proximity to the helmet viewing window in the helmet. However, in this apparatus a welder must still move their visual focus away from the welding work area in order to focus on the information located proximate to the welding window or the welder must accept the information peripherally while continuing to focus on the welding work area.

US 2015/209887 A1 discloses a welding helmet with voice controlled head up display. US 2016/125653 A1 discloses a welding helmet with voice controlled power settings for power supply, the welding helmet having a button.

### BRIEF SUMMARY OF THE INVENTION

The present invention relates to a welding system as defined in claim 1. Other embodiments of the present invention allow the user to change settings and/or operations of the welding system or welding power supply by using voice commands while not having to take off the helmet.

Various aspects will become apparent to those skilled in the art from the following detailed description and the accompanying drawings. Further embodiments are inferable from the description, drawings and claims.

### BRIEF DESCRIPTION OF THE DRAWINGS

The above and/or other aspects of the invention will be more apparent by describing in detail exemplary embodiments of the invention with reference to the accompanying drawings, in which:
FIG. 1 is a schematic view of a welding system according to the present invention;
FIG. 2 is an enlarged view of a welding helmet similar to the helmet of FIG. 1 including a camera;
FIG. 3 is a cross-sectional diagram of a welding helmet similar to the helmet of FIG. 2 including a projector;
FIG. 4 is a cross-sectional diagram of a welding helmet similar to the helmet of FIG. 3 including an integrated video display;
FIG. 5 is a perspective view of a welding helmet similar to the helmet of FIG. 2 including binocular cameras;
FIG. 6 is an interior view of a welding helmet similar to the helmet of FIG. 5 showing binocular viewing screens;
FIG. 7 is a cross-sectional diagram of an exemplary embodiment a welding helmet with a HUD;
FIG.8 is a schematic view of a welding system according to an exemplary embodiment of present invention;
FIG. 9 is an interior view of a welding helmet with a HUD;
FIGs. 10A and 10B are an interior views of a welding helmet with a HUD;
FIG. 11 is a cross-sectional diagram of an exemplary embodiment of a welding helmet with a HUD;
FIG. 12 illustrates exemplary views of information that can be displayed;
FIG. 13 illustrates a further embodiment of a welding system in accordance with an exemplary embodiment disclosed herein; and
FIG. 14 illustrates a further exemplary embodiment of a welding helmet.

### DETAILED DESCRIPTION OF EXEMPLARY EMBODIMENTS

Exemplary embodiments of the invention will now be described below by reference to the attached Figures. The described exemplary embodiments are intended to assist the understanding of the invention, and are not intended to limit the scope of the invention in any way. Like reference numerals refer to like elements throughout.

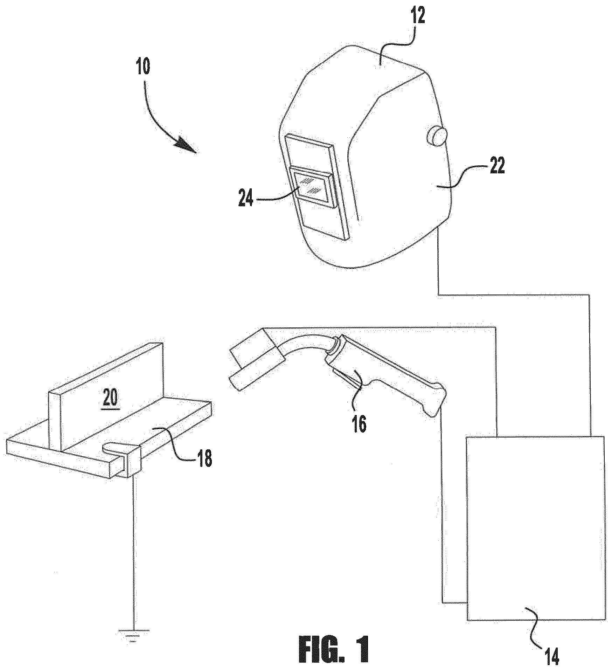

Referring now to the drawings, there is illustrated in FIG. 1 a welding system 10. The welding system 10 includes a welding helmet 12, a welding system 14, a welding gun 16 and a work piece 18. The work piece 18 generally defines a welding work area 20 where the welding gun 16 may be used to form a weld.

The welding system 14 includes welding equipment for generating a welding current and voltage, a welding control system for controlling the welding current and voltage, and a monitoring system for monitoring the welding current and voltage. That is, the welding system, can be on known or used welding power supply having a known construction and operation. The monitoring system may also monitor a variety of other operating parameters, such as but not limited to, wire feed speed, amount of wire used/amount of wire remaining, any type of welding feedback desired by the operator and any other desired operating parameter.

The welding helmet 12 includes a main body 22 with a visual display 24 connected to the main body 22. The display 24 may be a window including a welding lens, a video monitor, such as an LCD display or LED array, or any other device suitable to allow a welder to see the welding work area 20. It must be understood that in such an example where the display 24 is a video monitor video processing may be utilized to enhance the pictures of the welding operation. Further, recording devices may optionally be included in the display, for example, to record and later playback welding operations for analysis and/or evaluation.

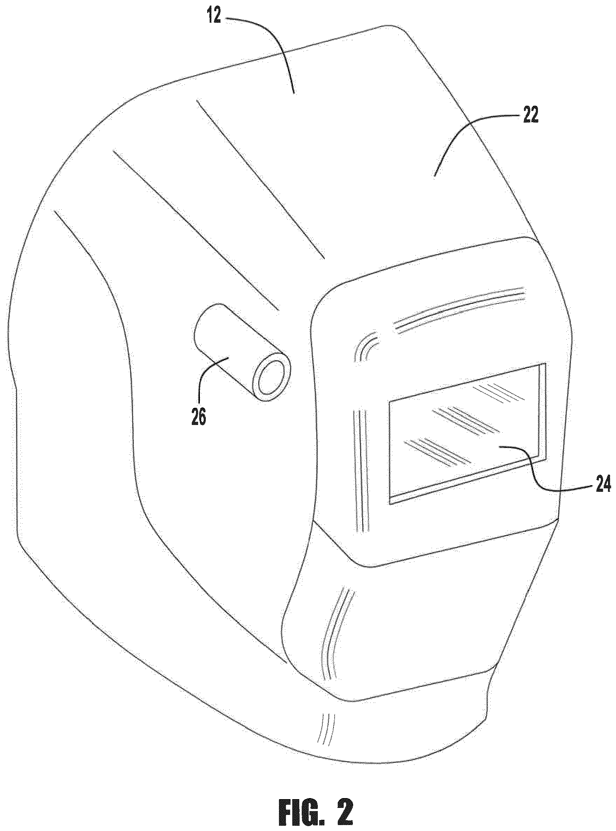

As shown in FIG. 2, a welding helmet 12 may include a camera 26 mounted at or proximate to the point of view of the welder. In the example where the visual display 24 is a video monitor, the camera 26 may provide video pictures of the welding work area 20 to the display 24. Further, the camera 26 can be used to record the welding operation as it is ongoing, so that the welding operation can be viewed at a later time.

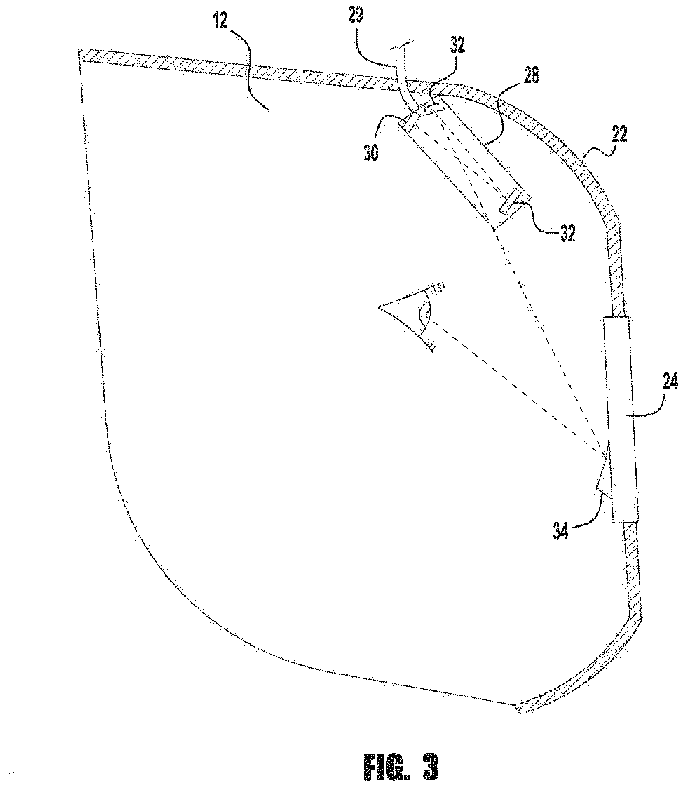

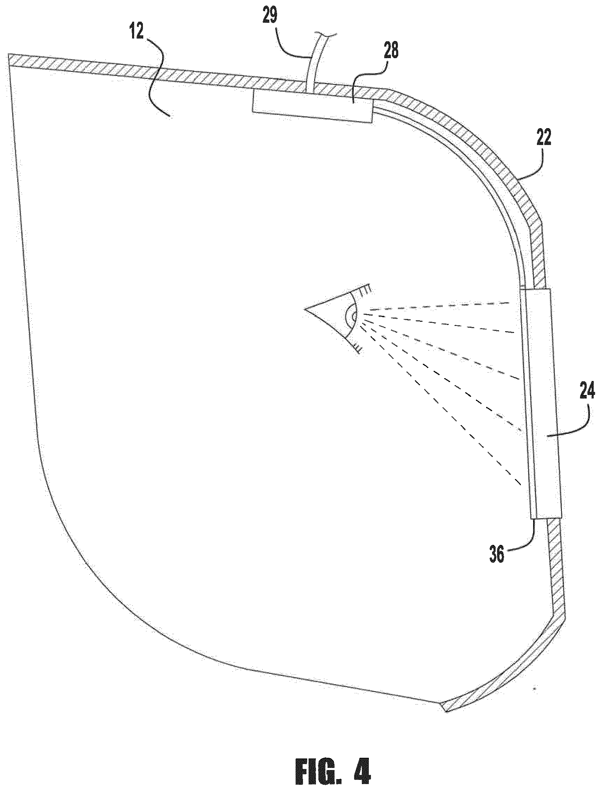

As shown in FIGS. 3 and 4 an information generating mechanism 28 is in communication with the monitoring system of the welding system 14 and capable of generating an image representative of information from the monitoring system based upon the monitored welding parameter, such as current and voltage upon the visual display 24 where the focus of the image is at a focus range (i.e., having a focal point) with an associated welding work area, e.g. outside of the main body 22 of the welding helmet 12. That is, the focal range of the image displayed in/on the display 24 is set to be at a range associated with the location of the welding work area 20. For example, the image may be symbolic, alpha-numeric, or any other device suitable to indicate the information. Thus, a welder may view an image representative of information about a welding operation without removing focus from the work area. Thus, in at least one embodiment the welder may focus on the work area and the image of information at the same time.

It must be understood that among other types of information, along with a variety of other parameter, the information based upon welding current and voltage includes, but is not limited to, welding current feedback, welding voltage feedback, control settings of the welding equipment, statistical information of the welding process, benchmarks or limits including capacity representations, alerts including material shortage or low flow, a representation of an intended or desired weld, etc.

Further, in one embodiment, the camera 26 is used to calibrate the depth of the image relative to the welding work area 20. This calibrated depth can be used to determine the focus of the information displayed on the display 24. For example, if the camera 26 determines that the distance from the helmet to the work area is 2 feet, the images and/or information shown on the display 24 is displayed such that the image has a focal point which would be at 2 feet beyond the helmet. As explained above, this allows the displayed information to be displayed at a same focal length as the weld area 20 so that the welder need not change his/her eye focus during a welding operation. In another embodiment, positions sensors on the welding gun may be used to calibrate the depth of the image. Such sensors can include, but are not limited to, magnetic sensors, optical sensors, acoustics sensors, and the like, which are sensed using an appropriate sensing system to allow for the positioning of the welding gun to be determined. This data can be used to aid in determining the focal range/distance of the work area relative to the helmet. In particular applications it is highly desirable to carefully align the image and the welding work such that the information represented in the image is easy for the welder to access and such that the information in the image is readily accepted by the welder.

In the example where the visual display 24 is a video monitor, information generating mechanism 28 may include an image representative of information from the monitoring system based upon the monitored parameter, such as welding current and voltage, in video pictures of the welding work area 20 shown on the display 24.

As indicated at 29, the information generating mechanism 28 may be in wired or wireless communication with other devices as desired.

In FIG. 3, the information generating mechanism 28 is a projector. The projector may, for example, include an internal LCD display or LED array 30 along with a number of associated mirrors 32 to reflect the image generated to the visual display 24. The reflected image gives the image the appearance of depth relative to the visual display 24 and thus puts the image at a focus range with an associated welding work area and outside of the main body 22 of the welding helmet 12 and optionally at the same focal distance as the associated welding work area 20. Optionally, a reflective surface 34 may be placed upon a portion of the visual display 24 in order to achieve a desired amount of reflection or reflection angle. In one embodiment, teleprompter type technology may be utilized to place the image upon the display 24 or surface 34. Additionally, it must be understood that one embodiment includes the use of an LCD display or other similar display within the helmet to generate the image which is then sent along an optical path, such as by reflection or fiber optics or any other suitable device to place the image display 24 or surface 34.

In FIG. 4, the information generating mechanism 28 includes a screen, film, or sheet 36 integrated into the visual display 24. The sheet 36 may be a semi- transparent LCD film, electro-optic film, or any other suitable medium for the information generating mechanism 28 to produce an image generated in the visual display 24. In one application, the information generating mechanism 28 may project a stereogram on the welding lens such that a welder's eyes will separately view the images to create the perception of depth and thus focus the image at a focus range with the associated welding work area 20 and outside of the main body 22 of the welding helmet 12.

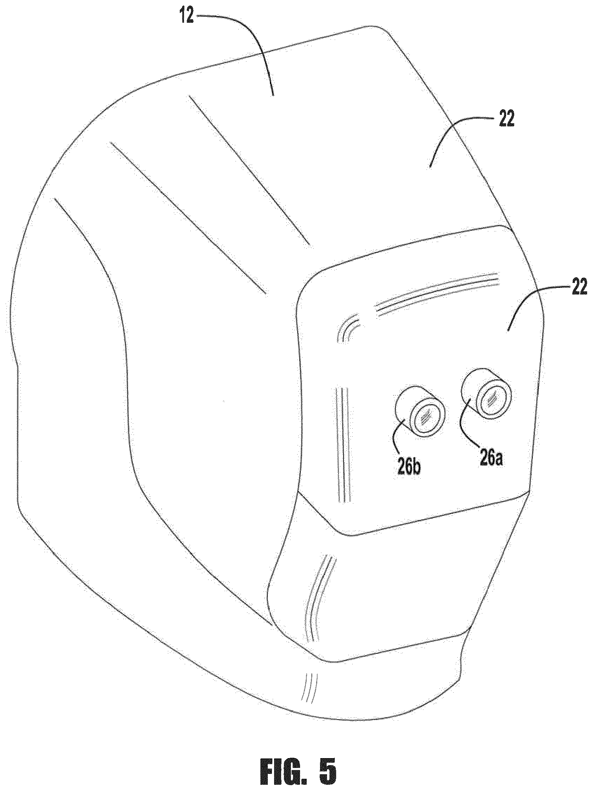

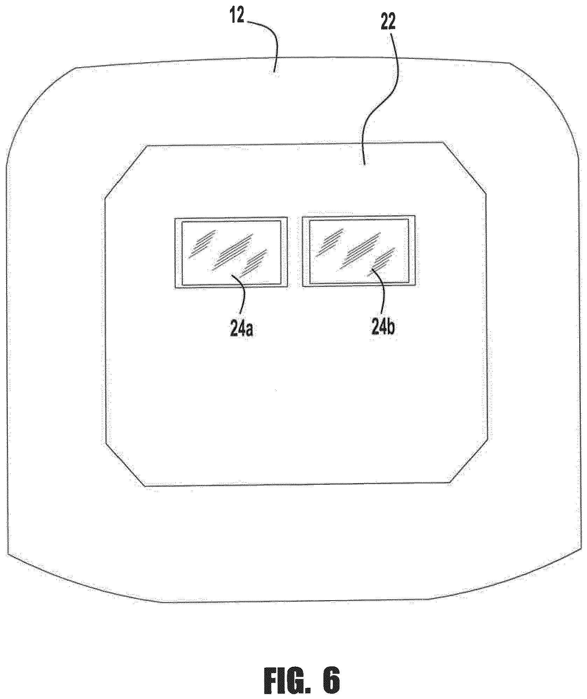

There is shown in FIG. 5 a welding helmet 12 including binocular cameras 26a and 26b. As shown in FIG. 6, these cameras 26a and 26b correspond to binocular viewing screens 24a and 24b. An information generating mechanism may produce an image to be generated in either of the viewing screens 24a or 24b or both. In one embodiment, the cameras 26a and 26b are placed in alignment with the screens 24a and 24b except on opposite sides of the main body 22, thus giving the welder the view directly in front of them. Additionally, in the embodiment with binocular cameras 26a and 26b and binocular viewing screens 24a and 24b the perception of depth of field is produced.

In any case, the image may be an overlay of text or graphics or video feedback. Additionally, it is contemplated that in at least one embodiment the system described above may be used in a remote welding situation, including but not limited to robotic welding or underwater welding.

While principles and modes of operation have been explained and illustrated with regard to particular embodiments, it must be understood, however, that this may be practiced otherwise than as specifically explained and illustrated without departing from its spirit or scope.

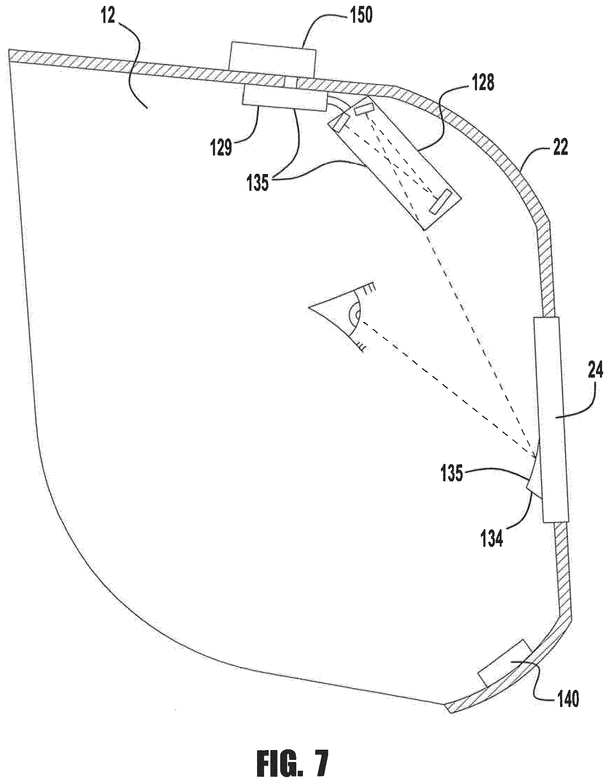

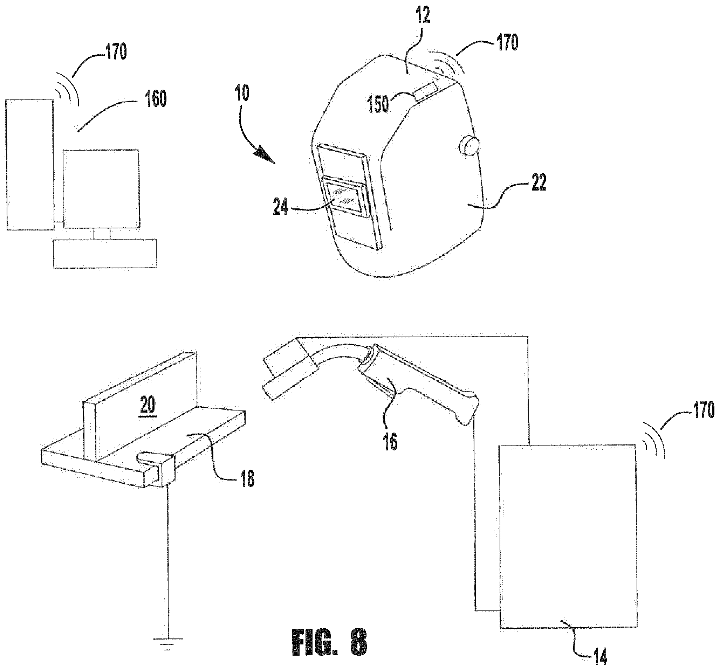

Some exemplary embodiments of the present invention, as illustrated in Figure 7, include a welding helmet 12 with a head-up display (HUD) 135 for the welder. In some embodiments, the HUD 135 includes a projector 128, a combiner 134, and an information generating device 129. The projector 128 can be, e.g., an LED array, an LCD display, a laser, a combination LED/LCD system, or some other suitable projector system. The projector 128 projects an image onto the combiner 134. The image can be in the form of text, graphics, video, etc. The projector 128 receives image information, e.g., in the form of a digital signal, from information generating device 129. The information generating device 129 generates and/or processes the image based on information received from an external source such as, e.g., a welding system 14 or a computer system 160 (see Figure 8). This information can include, among other types of information, welding parameters such as input power, input current, input voltage, welding voltage, welding current, welding power, tip-to-work distance, arc length, wire feed speed, etc. In some embodiments, the projector 128 and information generating device 129 can be integrated into a single physical unit. In some embodiments, the computer system 160 and/or the welding system 14 generates and/or processes the image and transmits the image information directly to projector 128, which can include and/or is connected to a wireless communication device.

The combiner 134 reflects the image projected from projector 128 to the welder. In some embodiments, light transmitted through lens 24 is also transmitted through combiner 134. Thus, the welder will see both the projected image and the field of view behind the combiner 134 at the same time. The light transmitted through lens 24 can be that from a welding arc transmitted through lens 24. In some embodiments, the lens 24 is of a type that rapidly and automatically changes from transparent to dark when the lens 24 detects that a welding arc has been initiated. The auto-darkening feature protects the welder's eyes from damage that could occur if the eye is exposed to the welding arc. The auto-darkening lens is transparent when no arc is detected and thus allows the welder to see the work space even when the welding helmet 12 is flipped down over the welder's face. With an auto-darkening lens, the light transmitted through lens 24 and combiner 134 can be either the light from the welding arc or normal room lighting depending on whether a welding operation is taking place.

In some embodiments, the combiner 134 collimates the reflected image such that the projected image appears to be at optical infinity. Thus, the welder will not have to refocus to see both the work space and the projected image - even during the welding process. In some embodiments, the combiner 134 is an appropriate transparent material, e.g., a flat piece of glass, that is angled such that the projected image from the projector 128 is reflected to the welder as illustrated in Figure 7. In some embodiments, the mounting of the combiner 134 to welding helmet 12 and/or lens 24 is such that the angle of reflection can be adjusted by the welder, as desired.

In some embodiments, the combiner 134 includes a coating that reflects monochromatic light from the projector 128. For example, the coating on the combiner 134 can be such that only, e.g., green light is reflected and all other light is transmitted through. Thus, the HUD 135 will provide the welder a transparent display that allows the welder to see information on the combiner 134 in green while still allowing the welder to view the work space. Of course, other coatings that reflect other colors or even multiple colors can be used on the combiner 134. For example, the combiner 134 can be coated such that it reflects the colors green and red. While in the normal operating range, the information, e.g., welding current, may be displayed in green and when outside the normal operating range, the information, e.g., welding current, can be displayed in red. The information provided to the welder can include welding operating parameters such as, e.g., input current, input voltage, input power, welding current, welding voltage, wire feed speed, contact tip-to-work distance, arc length, mode of operation, etc.

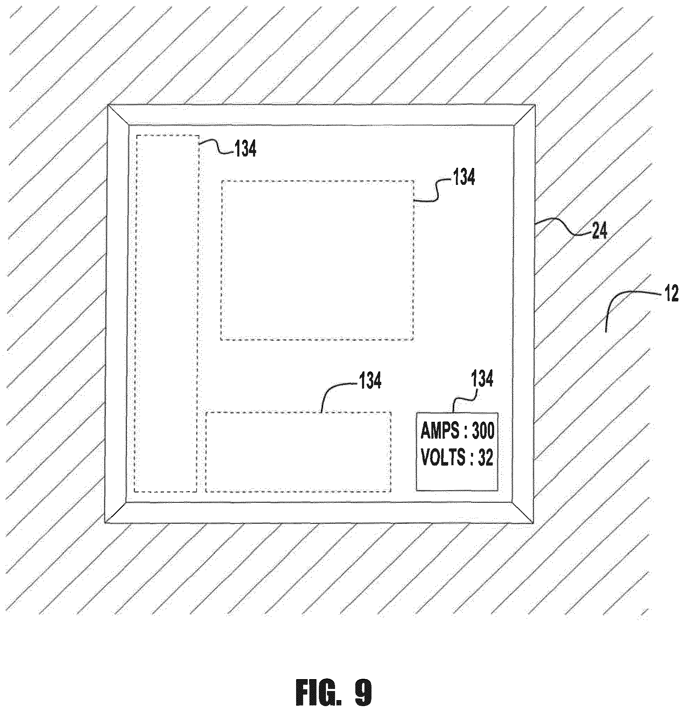

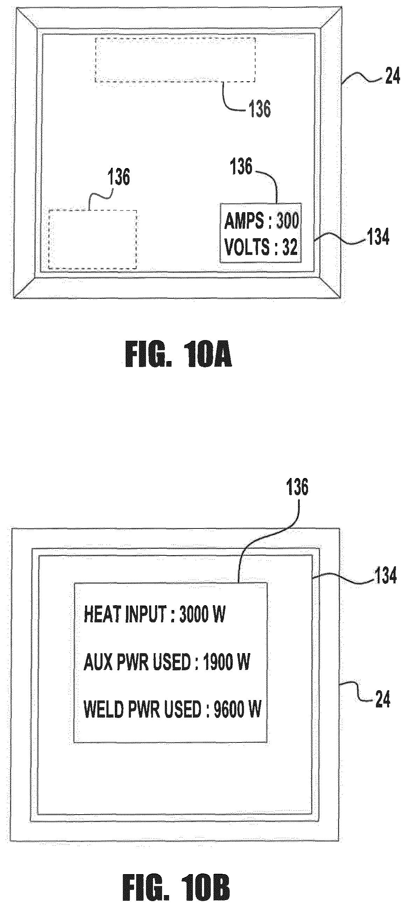

The size, shape, and placement of the combiner 134 relative to the lens 24 can vary, as desired. For example, Figure 9 illustrates various sizes, shapes, and locations for the combiner 134. The illustrated sizes and locations are exemplary and any appropriate size, shape, and location can be utilized. For example, in some embodiments, the combiner 134 is sized such that it covers the entire opening of lens 24 (see FIGs. 10A and 10B). Similarity, an image window, i.e. the window in which actual information is displayed, on the combiner 134 can be sized and/or located as desired. For example, as seen in Fig-ure 10 A, during an actual welding process an image window 136 can be displayed in a corner and/or along an edge of the combiner 134 so that the welder is not distracted but still has the information available, if desired. When the welding process is not occurring, the image window 136 can be displayed larger as seen in Figure 10B. The HUD 135 can be configured such that the image 136 is automatically resized based on whether welding system 14 is performing a welding operation. Alternately, or in addition, the HUD 135 can be configured such that the resizing of image 136 is a manual operation by the welder.

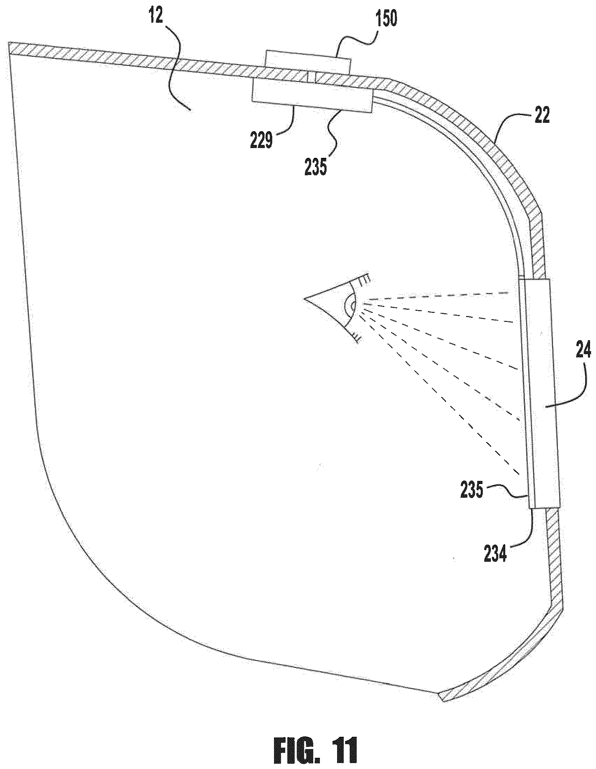

In some embodiments, the projector is not used. As illustrated in Figure 11, a HUD 235 includes combiner 234 and information generating device 229. In this exemplary embodiment, the image is produced directly in the combiner 234. The combiner 234 can be, e.g., an LCD display, optical waveguide, an electro-optical medium, or some other suitable medium for producing an image. Similar to combiner 134 discussed above, the size, shape, and placement of the combiner 234 relative to the lens 24 can vary, as desired, including having a display size that is equal to the size of the window of lens 24. In addition, similar to image window 136 discussed above, an image window on combiner 234 can be sized and/or located as desired.

The combiner 234 receives image information, e.g., in the form a digital signal, from information generating device 229, which generates and/or processes the image based on information received from welding system 14 and/or computer system 160. In some embodiments, the combiner 234 and information generating device 229 can be integrated into a single physical unit. In some embodiments, the combiner 234 and lens 24 can be integrated into a single physical unit. In some embodiments, the combiner 234, information generating device 229, and lens 24 can be integrated into a single physical unit. In some embodiments, the computer system 160 and/or the welding system 14 generates and/or process the image and transmits the image information directly to combiner 234, which can include or is connected to a wireless communication device.

The information generating devices 129 and 229 can each include a communication device 150 to communicate via, e.g., a wireless network 170 or a wired network with welding system 14 and/or computer system 160. The wireless network 170 can operate using, e.g., Bluetooth, WiFi (IEEE 802.11) or some other wireless protocol. In some embodiments, the welding system 14 can provide information such as e.g., input power, input current, input voltage, welding current, welding voltage, welding power, contact tip-to-work distance, arc length, wire feed speed, etc. in real- time to, e.g., aid the welder while the welding operation is going on. Alternatively, or in addition, the welding system 14 can send welding performance information after the welder has stopped welding. For example, the welding system 14 can transmit information such as, e.g., heat input, duration of welding, etc. after, e.g., the welder system 14 is turned off, indicating that the welder is done welding. Such information might be useful to the welder in order to make corrections before starting the next welding segment.

In some embodiments, the computer system 160 performs all the calculations such as, e.g., heat input, welding duration, etc. The computer system 160 can communicate with the welding system 14 and/or the welding helmet 12 via, e.g., wireless network 170 or a wired network. In some embodiments, the computer system 160 collects, stores, and/or analyzes information received from the welding system 14. In some embodiments, the computer system 160 transmits the image information to the welding helmet 12 instead of or in addition to the welding system 14. In some embodiments, the computer system is incorporated into or is integral to the welding system 14.

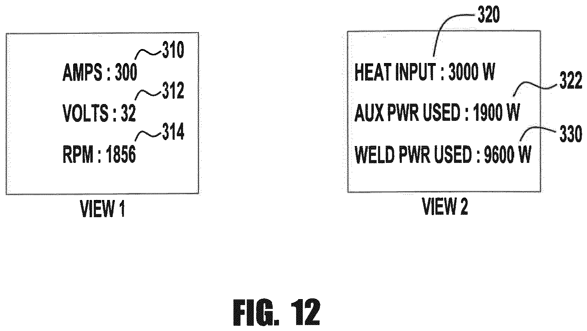

In some embodiments, the image information seen by the welder is configurable. For example, the computer system 160 and/or the welding system 14 can be configured with different "views" or image screens that the welder can select. For example, as illustrated in Figure 12, the welding information can be presented to the welder using several image screens or "views." View 1 can represent real-time operating parameters of an engine-driven welder such as, e.g., welder output amps 310, welder output volts 312, engine speed 314, etc. A second "view," View 2, can represent performance totals such as, e.g., welding heat input 320, Auxiliary Power Used 322, Welding Power Used 324, etc. In other exemplary embodiments, other data can be shown. For example, the HUD can display engine related information such as RPM, engine temperature, oil pressure, air compressor output pressure (if equipped), and any trouble codes from an engine control computer to allow the welder to be warned of any issues. The views can be customized to meet the welder's needs. For example, the views can be customizable based on the type of welding (TIG, MIG, etc.), material being welded (steel, aluminum, etc.), type of weld (fillet, butt joint, etc.), or on some other basis. In addition, the views can be customized for each welder. For example, after a welder identifies himself or herself by, e.g., logging into the computer system 160 or welding system 14, by using a token such as, e.g., a RFID tag, or by some other means, the computer system 160 and/or the welding system 14 can display a set of "views" that are specific to the welder, e.g., based on the welder's preferences, experience level, etc.

The welder can turn the HUD 135, 235 on and off and scroll through the "views" using controls (not shown) located on the welding helmet 12. Alternatively, or in addition, the welder can control the HUD 135, 235 using voice commands. The welding helmet 12 can include a microphone system 140 (see Figure 7) that picks up audio command from the welder. The microphone system 140 can then relay the audio commands to welding system 14 and/or computer system 160 using communication device 150. The welding system 14 and/or the computer system 160 interprets the commands and sends the appropriate instructions and information to the information generating device 129, 229. Information generating device 129, 229 will then control projector 128 and/or combiner 234 based on the received information and instructions. For example, a welder can say "SHOW CURRENT" and the system will display the welding current. Other voice commands can be used to allow the user to display the desired information. These voice commands can be used, prior to, during or after the completion of a welding process. In addition to the commands discussed above, the welder can adjust the size and location of the image window 136, the brightness of the image display, the color of the image display, etc. In some embodiments, the welder can control the opacity of the image to make the image more or less transparent, e.g., from nearly 100% transparent to 100% opaque. Along with welder adjustments, some display parameters such as, e.g., brightness, opacity, color, etc. can be adjusted automatically by at least one of information generating device 129, 229, computer system 160 and welding system 14 based on whether the welding arc is sensed and/or the level of ambient light in the room.

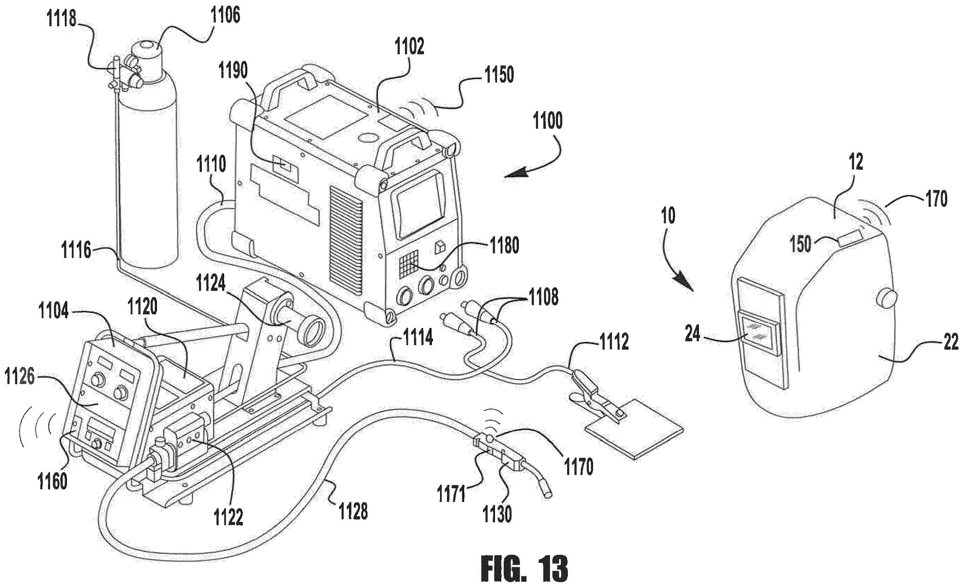

Figure 13 depicts a further exemplary embodiment of a welding system 1100 of the present invention, similar to that described above, the welding system 1100 includes a welding power source or power supply 1102, wire feeder 1104, and gas supply 1106. The welding power source 1102 includes power cables 1108, control cable 1110, and power supply cable (not shown). Power cables 1108 include a ground wire and clamp 1112 connected to a work piece and power cable 1114 configured to connect to wire feeder 1104. Control cable 1110 may be configured to connect to wire feeder 1104. In another embodiment (not shown), the control cable 1110 may be configured to be wireless, similar to the wireless technology discussed above. It is understood that welding power source 1102, power cables 1108, and control cable 1110 can have any configuration suitable for supplying power and welding controls to the welding system 1100. As with the embodiments above, the power supply 1102 and the system 1100 overall can be configured consistent with known welding systems. Because such systems are known, the detailed discussion of their operation is not further discussed herein.

Further illustrated in Figure 13, is gas conduit 1116 and regulator 1118, which are configured to connect the gas supply 1106 to the wire feeder 1104. Gas supply 1106 may include inert gases, active gases, or a combination of both, including but not limited to argon, helium, carbon dioxide, argon and helium, argon and hydrogen, and other gas combinations. In another embodiment (not shown), welding apparatus 1100 uses welding wire that is coated with a material that forms a gas shield when burned, therefore, a gas supply may not be necessary in all embodiments. It is understood that the gas supply may be any gas or combination of gases configured to shield a weld from the atmosphere.

As shown in Figure 13, the wire feeder 1104 may include a housing 1120, gear box 1122, wire spool assembly 1124, and user interface 1126. Extending from gear box 1122 is a hose 1128 that is configured to connect to welding gun 1130. Housing 1120 may be connected to user the interface 1126 and gear box 1122. Further, the control cable 1110 and power cable 1114 extending from welding power source 1102 and gas conduit 1116 extending from gas supply 1106 are configured to connect to housing 1120, gear box 1122, and hose 1128. Gear box 1122 includes at least a plurality of rollers (not shown) that advance and retract the welding wire (not shown) and a wire guide (not shown) that controls the path of the welding wire. It is understood that wire feeder 1104 may have any configuration suitable for receiving a gas supply, a power supply, and welding controls.

Extending between gear box 1122 and welding gun 1130 is hose 1128 which operatively connects the welding wire and wire conduit, a gas line (if required), and a welding gun trigger switch connection. In another embodiment (not shown), as discussed above, hose 1128 does not include a gas line. In yet another embodiment (not shown), hose 1128 may include a control cable configured to connect welding gun 1130 to at least one of the following: welding power source 1102, wire feeder 1104, and gas supply 1106. Hose 1128 can be any diameter and length configured to contain the welding wire, the gas hose, and the switch connection. Hose 1128 is made of any material suitable for welding environments. It is understood that hose 1128 and welding gun 1130 may have any configuration suitable for supplying welding wire, welding gas, and controls through the hose and to the welding gun.

In the illustrated embodiment of welding system 1100, user interface 1126 includes at least one of the following: a control system, a computer (or optionally at least a CPU) with sufficient processing capabilities to implement and/or run a software program and a setup program and control the operation of the embodiments described herein. The helmet 10 can be configured like any of the embodiments discussed herein. As shown, the helmet 10 can have wireless communication ability 170 via any known wireless communication technology. Further, each of the wire feeder 1104 and the power supply 1102 can have wireless communication devices 1160 and 1150, respectively, which allow for wireless communication between the respective components. Of course, communication can also occur over a wired connection as well between the helmet 10 and the wire feeder 1104 and/or power supply 1102. Further, in other exemplary embodiments, the torch or welding gun 1130 can have a wireless communication module or device 1170 which communicates with the wireless device 170 on the helmet 10. For example, in embodiments where there welding operation is far away from the wire feeder 1104 and/or power supply 1102 the helmet 10 can communicate wirelessly with the torch 1130 (for example, via Bluetooth technology) and those communications and any transferred information can be sent over communication lines in the conduit 1128 which can then be received by the wire feeder 1104 and/or the power supply 1102 via known communication protocols. This exemplary system 1100 and its coupled communications can allow the user to use voice commands to control an operation and settings of the system 1100. This is described more fully below.

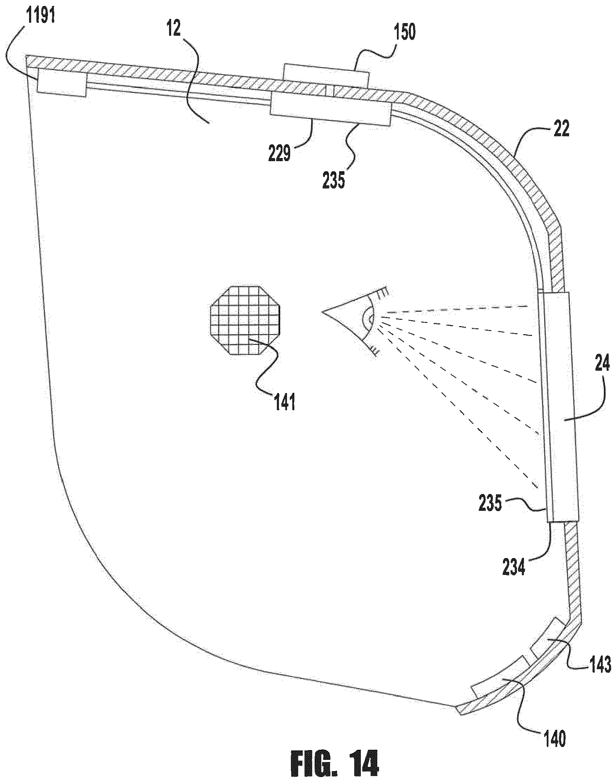

As previously, described, any and all embodiments of the present invention have a helmet with a microphone 140 (Figure 7 and Figure 14). The microphone 140 can be positioned at a location which picks up the welder's voice instruction and/or command. Using the various communication methodologies disclosed herein the voice commands are sent to the wire feeder 1104 and/or power supply 1102 as needed. Using this, the welder can control both the information displayed and the settings and operation of the system 1100. In addition to a microphone, the helmet 10 can include at least one speaker 141 (Figure 14) which can provide audio cues and triggers to the welder when using the system 1100. The speaker(s) 141 can be mounted within the helmet and positioned near the ear of a user when the helmet is worn. Like the microphone, the speaker(s) 141 can be coupled to the communication components of the system to ensure the desired audio signals and data communicated to the welder wearing the helmet. In yet a further exemplary embodiment, the helmet can include a "white noise" generator 143, or other type of noise cancelation device which is capable of reducing or interfering with the ambient noises created in a welding environment, the sound interference device 143 can be used to allow for improved audio communication to the welder via the speaker 141 or can acts as a sound filtering device to allow for easier voice recognition by the microphone 140. For example, if the welder is conducting a welding operation the ambient noise can be high - causing voice recognition issues by the control electronics of the system 1100. Thus, the filter device/noise cancellation device 143 can be used to filter out the ambient welding noises so that the welders commands/instructions are clearly received and recognized. This, and other aspects of these embodiments will be discussed more fully below.

As described in previous embodiments, the welder can change the display using voice commands. However, in other exemplary embodiments, using similar technology (for example, voice recognition technology), the welder can change welding operation settings. That is, while the welder is remote from the power supply 1102 and/or the wire feeder 1104, and while wearing the helmet, the welder can cause welding settings, parameters, and welding operations to be changed via the use of voice commands. In exemplary embodiments, the controllers for at least one of, or both, of the wire feeder 1104 and the power supply 1102 contain voice recognition software and capabilities to allow the welder's voice to control the operation and settings of the system as described herein. Such software and systems are generally known and need not be described in detail herein. For example, similar voice recognition systems exist in the customer service industries, where users use audio/verbal commands over a phone to obtain information, etc. Such technology, as well as other similar technology can be used in embodiments described herein.

Further, as shown in Figure 13, the present invention utilize a push-to-talk switch 1171 on the torch 1130 which enables the user/welder to begin the audio control/changing of the welding settings. Such arrangement according to the present invention prevents a user from inadvertently changing a setting. For example, prior to making a setting change, a welder engages the button/switch 1171. This sends a signal to at least one or, or both, of the power supply 1102 or the wire feeder 1104 to be ready to receive an audio command. After the switch 1171 is engaged the welder can make the voice command - for example - "change current to 300". After completion of the audio command the user/welder then disengages the switch 1171 of simply press the switch again (depending on its operation) indicating that the audio command has ended. In other exemplary embodiments according to the present invention, no separate switch is used, but the trigger on a MIG torch is used to initiate a setting change operation. For example, the system can be configured such that 2 or more quick actions on the torch trigger can signal that an audio instruction is to be initiated, and after the audio instruction, the trigger is again depressed quickly for a number of times to indicate the end of the instruction. Further, either the switch 1171 or the torch trigger can be used to verify a proper setting change. That is, according to the present invention, either the switch 1171 or the torch trigger is used by the welder to confirm that a setting is correct to verify the setting. In other exemplary embodiments, the torch 1130 can have both a switch 1171 and trigger and the activation and/or depression of each simultaneously can be used to initiate, end and/or verify a setting or audio instruction.

Of course, in other exemplary embodiments, the system 1100 can be set up such that certain audio cures or words can be used to initiate, end or verify settings and/or setting changes. For example, to initiate a change a welder can say the word/phrase "CHANGE CURRENT". This is recognized by a controller in either (or both) of the wire feeder 1104 and power supply 1102 as an audio cue which is used to initiate a change for the welding current. At that time, the system 1100 can cause a visual cue to be displayed in the helmet to the welder (for example, as described in the embodiments above) - such as "CHANGE CURRENT - NEW CURRENT LEVEL?" - after which the user would verbally state the new current setting - e.g., "300". The system 1100 can then cause the new current level to be displayed as "300" and ask the welder to "VERIFY NEW CURRENT" (or something similar). Then the user/welder can engage a switch 1171, the gun trigger, or whatever other mechanism is used for verification of the new setting. For example, in some embodiments, the system can recognize a certain audio signal as verification. For example, the system can recognize the word "VERIFIED" (spoken through the microphone 140) as a signal indicating that the signal is correct. Of course, in exemplary embodiments the system 1100 also has the capability to make corrections when the audio commands where not properly recognized. In such systems the user can again use the switch 1171, trigger and/or audio signals to make corrections. For example, if the user initiated a desired current change to 300 amps, but the system displayed a new current of "400 Amps" the user could use either audio commands or a switch/trigger initiation to indicate that this new setting as incorrect. For example, the welder could say "INCORRECT" at which time the system will re-start the current setting protocol until the welder verifies that the new setting is correct.

As shown in Figure 13, in some exemplary embodiments, the power supply 1102 has a speaker/microphone system 1180. This can allow a user at the power supply 1102 to communicate with the welder wearing the helmet and vice versa. The speaker/microphone system 1180 can have a push to talk switch and/or a visual indicator, such as a light, etc. This will allow the welder to easily communicate with someone near the power supply 1102 when the welder is positioned remotely and without removing the helmet. In exemplary embodiments, the visual indicator can illuminate when the welder triggers the switch 1171, the gun trigger, or any other mechanism when the welder wants to communicate with someone near the power supply 1102. For example, when the welder wants to communicate with someone, he/she presses the switch 1171, which causes an illumination and/or an audio signal at the power supply 1102 so that someone nearby can interact/respond. Of course, in other exemplary embodiments, the speaker/microphone system 1180 can also be installed on the wire feeder 1104, or any other component of the welding system.

In further exemplary embodiments, the system 1100 permits helmet-to- helmet communications. That is, in some embodiments the communication networks described herein can allow communication between helmets. This can be done wirelessly or via a wired connection. For example, a plurality of power supplies 1102 can be networked to allow communication with each other and the welder which is using each power supply, respectively.

In additional exemplary embodiments, the voice modification system can be "locked-out" when the system is welding. That is, the controller/control system of the power supply 1102 and/or wire feeder 1104 will not accept and/or will not allow any voice command changes to parameter settings while the power supply 1102 is outputting a welding current (or a plasma cutting current if the system is a plasma cutting system). However, in other exemplary embodiments, where it may be beneficial to change settings while welding, the system 1100 can allow setting changes while welding. In such systems, the audio recognition system internal to the power supply 1102 and/or wire feeder 1104 would use a specific audio signal/indication/word to allow for modification of a parameter. Such an audio signal should be distinct from normal welding sounds to ensure avoidance of an inadvertent parameter change. For example, the control system could use a trigger word/phrase such as "PARAMETER CHANGE", which the system 1100 would recognize as requiring a change while welding. When the appropriate audio signal is recognized by the controller, the system will allow a change, for example of wire feed speed, while the welding operation is ongoing. In some exemplary embodiments only a subset of parameters can be changed while welding. This ensures that certain parameters, for which on-the-fly change is not desired will remain locked out. For example, when welding the system 1100 may permit on-the-fly WFS changes, but no voltage setting changes.

An exemplary method of using the system is described below. Of course, the following is intended to merely exemplary and not intended to limit the overall scope of the embodiments described herein.

Prior to a welding (or cutting operation) a welder/user puts on the helmet 10 and can use a trigger signal to indicate to the system 1100 that an audio command is coming. For example, the user can activate the switch 1171. Upon receiving this indication the controller/CPU (or like component) in the power supply 1102 and/or the wire feeder (1104) can cause either, or both, of a visual indication in the helmet (see discussions above) and an audio cue (via the speaker 141) to indicate that the system 1100 is ready to modify/set parameters. Then the user can provide a further command, such as "SET CURRENT," "SET WIRE FEED SPEED," "SET VOLTAGE," "SET SHIELD GAS", etc. The system controller recognizes these commands and causes an appropriate visual and/or audio signal to be relayed to the helmet 10. For example, the system can display "SETT CURRENT" and/or provide an audio recording of "SET CURRENT" to indicate to the user that the system is ready for the setting of the current. After this confirmation, the user can then provide an audio indication of the current setting. For example, the user can say "350" or "3 - 5 - 0", or whatever desired audio protocol is desired. The system 1100 then recognizes this information and can visually display and/or provide an audio confirmation to confirm the proper setting. For example, the system 1100 can visually display "CURRENT SET 350" and/or provide an audio confirmation via the speaker 141. If the displayed setting is correct then the user can either use a switch/trigger on the gun to indicate that the setting is appropriate or provide an audio confirmation such as "VERIFIED", "CORRECT," "SET", etc. With this verification the power supply 1102 will make the appropriate setting for the operation. However, if the setting is not correct then the user can use either the switches and/or audio signals to correct the setting. For example, the user can restate the setting - for example "CURRENT SET 350" - and repeat this as needed until the proper setting is displayed. In other embodiments, the user can state "CORRECT CURRENT" or can say "NOT CORRECT" and then repeat the setting process. The user can thus use the above protocol, or something similar to set all operation parameters, including: current, voltage, wire feed speed, shielding gas pressure, shield gas type, etc. The user can also use audio commands to set the welding process type. For example, pulse, CC, CV, STT, etc. Once the appropriate parameters are set for a given welding/cutting operation, the user can activate a switch/trigger and/or provide an audio signal that the parameters are set and appropriate for a given operation. For example, the user can say "PARAMETERS SET", "EXECUTE", etc. to indicate to the system that all parameters are set. Once this is done, some exemplary embodiments can provide a visual and/or audio indication to user in the helmet to indicate that the system 1100 is ready to weld. For example, a green light can be shown, along with the parameter settings, or a visual and/or audio indication saying "READY TO WELD" or something similar can be provided to the user, after which the welding operation can be begin. As indicated above, in some embodiments, the parameters cannot be changed until the welding operation is stopped, whereas in other embodiments, at least some of the parameters can be changed during welding.

The system and helmet can be set by a user to allow the voice commands to control a desired specific function. That is, prior to welding, the user can use voice commands or other inputs at the helmet and/or a user input at the power supply to select a welding function/operation that will be controlled with voice commands. This welding parameter can include current, wire feed speed or voltage, for example. Of course other desired controllable parameter can be selected as needed depending on the welding operation. Once the controllable parameter is selected then the user can use simple voice commands in the helmet to control this selected parameter. For example, for a TIG type welding operation, the user can select the current to be the controllable parameter. Then during welding the user can simply say "more" or "less", or "up" or "down", or "increase" or "decrease." This simple voice commands will be used as described in the embodiments above to control the selected parameter and will move the current (or selected parameter) accordingly. Similarly, in other desired welding operations the wire feed speed or voltage can be controlled in a similar manner. In exemplary embodiments, the power supply can have a voice command parameter user input device, such as a knob, switch, or other user input entry mechanism which allows the user to select the voice controlled parameter prior to welding. For example, as shown in Figure 13, the display screen shown on the face of the power supply 1102 can be an input device which use touch sense technology (such user inputs are known) which allows a user to select/input the parameter to be controlled. Once the selection is made the system will then use the voice commands (example above) to control the selected parameter. In this embodiment, the user would not have to say "Current," etc. before controlling the parameter, as it will have been preselected. Such an embodiment can be used with or without a visual display within the helmet. In further embodiments, the user can control the selected parameter with voice commands as described in previous embodiments.

In further exemplary embodiments the electronics on the helmet contain a battery (not shown) to power the electronic components on the helmet (described above) where the battery can be charged on the power supply 1100. That is, the helmet can have an electrical charging connector 1191 (see Figure 14) which is coupled to a battery (not shown) that can be in the device 229 for example. When the energy level of the battery is low the helmet can be mounted on a charging bracket 1190 on the power supply 1100 (see Figure 13). The charging bracket 1190 and helmet/connector 1191 are configured such that they can engage with each other and when coupled electrical power from the power supply 1100 is provided to the battery in the helmet to charge the battery. The electrical charging connection can be any known type including USB, etc. Further, it is known to provide auxiliary and low power from a welding power supply so aspects of that technology need not be described herein. This allows the helmet electronics to be charged at the power supply and increases the overall flexibility of the system. The battery (which can be either rechargeable or simply a replaceable battery) can be mounted anywhere on said helmet so as to not interfere with the user during welding.

While the invention has been particularly shown and described with reference to exemplary embodiments thereof, the invention is not limited to these embodiments. It will be understood by those of ordinary skill in the art that various changes in form and details may be made therein without departing from the scope of the invention as defined by the claims.

**REFERENCE NUMERALS**

| | | | |
|---|---|---|---|
| 10 | welding system | 234 | combiner |
| 12 | welding helmet | 235 | HUD |
| 14 | welding system | 310 | welder output amps |
| 16 | welding gun | 312 | welder output volts |
| 18 | work piece | 314 | engine speed |
| 20 | work area | 320 | welding heat input |
| 22 | main body | 322 | Auxiliary Power Used |
| 24 | visual display | 324 | Welding Power Used |
| 24a | viewing screen | 1100 | welding system |
| 24b | viewing screen | 1102 | power supply |
| 26 | camera | 1104 | wire feeder |
| 26a | camera | 1106 | gas supply |
| 26b | camera | 1108 | power cable |
| 28 | mechanism | 1110 | control cable |
| 30 | LED array | 1112 | clamp |
| 32 | mirror | 1114 | power cable |
| 34 | surface | 1116 | gas conduit |
| 36 | sheet | 1118 | regulator |
| 128 | projector | 1120 | housing |
| 129 | device | 1122 | gearbox |
| 134 | combiner | 1124 | wire spool assembly |
| 135 | head-up display (HUD) | 1126 | user interface |
| 136 | image | 1128 | hose |
| 140 | microphone system | 1130 | welding gun |
| 141 | speaker(s) | 1150 | communication device |
| 143 | generator | 1160 | communi cation device |
| 150 | device | 1171 | push-to-talk switch |
| 160 | computer system | 1180 | speaker/microphone system |
| 170 | wireless network | 1190 | charging bracket |
| 229 | device | 1191 | electrical charging connector |

## Claims

1. A welding system (10) comprising:
a welding power supply system which supplies a welding power to a welding torch (16) for welding a work piece (18); and
a welding helmet (12) to be worn by a welder during a welding operation with said welding power supply system, where said welding helmet (12) is in communication with said welding power supply system, the welding helmet (12), comprising:
a main body portion;
a visual display portion which is coupled to the main body portion, where said display portion allows a welding operation to be viewed with said display portion;
an information generating mechanism which generates an image to be displayed on said visual display portion said that said image is viewable during said welding operation;
a communication device coupled to said information generating mechanism which receives information to be displayed in said image;
a microphone coupled to said communication device, where said microphone is positioned such that said microphone receives audio instructions from a user of said helmet (12) when wearing said helmet (12),
wherein said communication device communicates said audio instructions received from said microphone to a welding power supply;
**characterized by**
a switch (1171) mounted on a welding torch (1130) or a torch trigger which activates said microphone to allow said communication device to receive signals from said microphone and where said image displays setting information from said welding power supply system.

2. The welding system of claim 1, further comprising a switch which activates said microphone to allow said communication device to receive signals from said microphone.

3. The welding system of claim 1 or 2, wherein said information generating mechanism generates visual confirmation of audio instructions received by said microphone.

4. The welding system of one of the claims 1 to 3, wherein said helmet (12) further comprises a battery and a charging connector (1191) through which electrical energy passes to charge said battery when said charging connector (1191) is coupled to an energy source.

5. The welding system of one of the claims 1 to 4, wherein said communication device is a wireless communication device.

6. The welding system of one of the claims 1 to 5, further comprising a speaker in said helmet which is coupled to said communication device where said speaker provides audio signals to said user.

7. The welding system of claim 6, wherein said audio signals are provided from said welding power supply.

8. The welding system of one of the claims 1 to 7, wherein the power supply system is configured not to accept and/or allow any voice command changes to parameter settings while the power supply is outputting welding current or a plasma cutting current.

9. The welding system of one of the claims 1 to 8, wherein the power supply system is configured to allow setting changes while welding, wherein a specific audio signal/indication/word is used to allow for modification of a parameter, such specific audio signal/indication/word being district from normal welding sounds to ensure avoidance of an inadvertent parameter change.

10. The welding system of one of the claims 1 to 9, wherein said information generating
mechanism generates visual confirmation of audio instructions received from said welding power supply; and/or
where said helmet further comprises a noise generator to interfere with ambient noise within said helmet; and/or
where said power supply further comprises a microphone and a speaker to allow audio communication from said welding power supply to said helmet.

11. The welding system of one of the claims 1 to 10, wherein output settings of said welding power supply are changed by using said audio instructions received by said microphone.

12. The welding system of one of the claims 1 to 11, wherein said communication device couples, with a communication link, with a second communication device in a second welding helmet such that audio communication between said helmet and said second welding helmet occurs.

13. The welding system of one of the claims 1 to 12, where said setting information is set on said welding power supply based on said audio instructions.

14. The welding system according to one of the preceding claims, comprising:
a welding parameter selection device for selected a controllable welding parameter;
wherein said audio instructions include a first instruction which increases said controllable welding parameter and a second instruction which decreases said controllable welding parameter, and
wherein said welding power supply increases said controllable welding parameter when said first instruction is received and decreases said welding parameter when said second instruction is received.

## Patentansprüche

1. Schweißsystem (10), das umfasst:
ein Schweißstromversorgungssystem, das einem Schweißbrenner (16) Schweißstrom zum Schweißen eines Werkstücks (18) zuführt; und
eine Schweißerhaube (12), die von einem Schweißer während eines Schweißvorgangs mit dem Schweißstromversorgungssystem zu tragen ist, wobei die Schweißerhaube (12) mit dem Schweißstromversorgungssystem kommuniziert, wobei die Schweißerhaube (12) umfasst:
einen Hauptkörperabschnitt;
einen visuellen Anzeigeabschnitt, der mit dem Hauptkörperabschnitt verbunden ist, wobei der Anzeigeabschnitt es erlaubt, einen Schweißvorgang mit dem Anzeigeabschnitt zu betrachten;
einen Informationsgenerierungsmechanismus, der ein auf dem visuellen Anzeigeabschnitt anzuzeigendes Bild generiert, so dass das Bild während des Schweißvorgangs betrachtet werden kann;
eine Kommunikationsvorrichtung, die mit dem Informationsgenerierungsmechanismus gekoppelt ist und Informationen empfängt, die in dem Bild angezeigt werden sollen;
ein Mikrofon, das mit der Kommunikationsvorrichtung gekoppelt ist, wobei das Mikrofon so positioniert ist, dass das Mikrofon Audioanweisungen von einem Benutzer der Haube (12) empfängt, wenn dieser die Haube (12) trägt, wobei die Kommunikationsvorrichtung die von dem Mikrofon empfangenen Audioanweisungen an eine Schweißstromversorgung übermittelt;
**gekennzeichnet durch**
einen Schalter (1171), der an einem Schweißbrenner (1130) oder einem Brennerauslöser montiert ist, der das Mikrofon aktiviert, damit die Kommunikationsvorrichtung Signale von dem Mikrofon empfangen kann, und wobei das Bild Einstellinformationen von dem Schweißstromversorgungssystem anzeigt.

2. Schweißsystem nach Anspruch 1, das des Weiteren einen Schalter umfasst, der das Mikrofon aktiviert, damit das Kommunikationsgerät Signale von dem Mikrofon empfangen kann.

3. Schweißsystem nach Anspruch 1 oder 2, wobei der Informationsgenerierungsmechanismus eine visuelle Bestätigung der durch das Mikrofon empfangenen Audioanweisungen generiert.

4. Schweißsystem nach einem der Ansprüche 1 bis 3, wobei die Haube (12) des Weiteren eine Batterie und einen Ladeanschluss (1191) umfasst, durch den elektrische Energie zum Laden der Batterie fließt, wenn der Ladeanschluss (1191) mit einer Energiequelle gekoppelt ist.

5. Schweißsystem nach einem der Ansprüche 1 bis 4, wobei die Kommunikationsvorrichtung eine Drahtloskommunikationsvorrichtung ist.

6. Schweißsystem nach einem der Ansprüche 1 bis 5, das des Weiteren einen Lautsprecher in der Haube umfasst, der mit der Kommunikationsvorrichtung gekoppelt ist, wobei der Lautsprecher Audiosignale an den Benutzer ausgibt.

7. Schweißsystem nach Anspruch 6, wobei die Audiosignale von der Schweißstromversorgung bereitgestellt werden.

8. Schweißsystem nach einem der Ansprüche 1 bis 7, wobei das Stromversorgungssystem so konfiguriert ist, dass es keine Änderungen der Parametereinstellungen per Sprachbefehl akzeptiert und/oder zulässt, während die Stromversorgung Schweißstrom oder Plasmaschneidstrom ausgibt.

9. Schweißsystem nach einem der Ansprüche 1 bis 8, wobei das Stromversorgungssystem so konfiguriert ist, dass es Einstellungsänderungen während des Schweißens erlaubt, wobei ein spezifisches Audiosignal/ein spezifischer Hinweis/ein spezifisches Wort verwendet wird, um die Modifizierung eines Parameters zu erlauben, wobei sich ein solches spezifisches Audiosignal/ein solcher spezifischer Hinweis/ein solches spezifisches Wort von den normalen Schweißgeräuschen unterscheidet, um zu gewährleisten, dass eine unbeabsichtigte Parameteränderung vermieden wird.

10. Schweißsystem nach einem der Ansprüche 1 bis 9, wobei der Informationsgenerierungsmechanismus eine visuelle Bestätigung der von der Schweißstromversorgung empfangenen Audioanweisungen generiert; und/oder
wobei die Haube des Weiteren einen Geräuschgenerator umfasst, um die Umgebungsgeräusche innerhalb der Haube zu stören; und/oder
wobei die Stromversorgung des Weiteren ein Mikrofon und einen Lautsprecher umfasst, um eine Audiokommunikation von der Schweißstromversorgung zu der Haube zu erlauben.

11. Schweißsystem nach einem der Ansprüche 1 bis 10, wobei die Ausgabeeinstellungen der Schweißstromversorgung unter Verwendung der durch das Mikrofon empfangenen Audioanweisungen geändert werden.

12. Schweißsystem nach einem der Ansprüche 1 bis 11, wobei sich die Kommunikationsvorrichtung - mit einem Kommunikationslink - mit einer zweiten Kommunikationsvorrichtung in einer zweiten Schweißerhaube so koppelt, dass eine Audiokommunikation zwischen der Haube und der zweiten Schweißerhaube stattfindet.

13. Schweißsystem nach einem der Ansprüche 1 bis 12, wobei die Einstellinformationen in der Schweißstromversorgung auf der Grundlage der Audioanweisungen eingestellt werden.

14. Schweißsystem nach einem der vorangehenden Ansprüche, das des Weiteren umfasst:
eine Schweißparameter-Auswahlvorrichtung zum Auswählen eines steuerbaren Schweißparameters;
wobei die Audioanweisungen eine erste Anweisung enthalten, die den steuerbaren Schweißparameter erhöht, und eine zweite Anweisung enthalten, die den steuerbaren Schweißparameter verringert, und wobei die Schweißstromversorgung den steuerbaren Schweißparameter erhöht, wenn die erste Anweisung empfangen wird, und den Schweißparameter verringert, wenn die zweite Anweisung empfangen wird.

## Revendications

1. Système de soudage (10) comprenant :
un système d'alimentation en énergie de soudage qui fournit une énergie de soudage à un chalumeau soudeur (16) pour souder une pièce d'ouvrage (18) ; et
un casque de soudage (12) à porter par un soudeur au cours d'une opération de soudage avec ledit système d'alimentation en énergie de soudage, dans lequel ledit casque de soudage (12) est en communication avec ledit système d'alimentation en énergie de soudage, le casque de soudage (12) comprenant :
une portion de corps principal ;
une portion d'affichage visuel qui est couplée à la portion de corps principal, dans lequel ladite portion d'affichage permet de visualiser une opération de soudage avec ladite portion d'affichage ;
un mécanisme de génération d'informations qui génère une image à afficher sur ladite portion d'affichage visuel de sorte que ladite image soit visualisable au cours de ladite opération de soudage ;
un dispositif de communication couplé audit mécanisme de génération d'informations qui reçoit des informations à afficher dans ladite image ;
un microphone couplé audit dispositif de communication, dans lequel ledit microphone est positionné de sorte que ledit microphone reçoive des instructions audio depuis un utilisateur dudit casque (12) lorsqu'il porte ledit casque (12),
dans lequel ledit dispositif de communication communique lesdites instructions audio reçues depuis ledit microphone à une alimentation en énergie de soudage ;
**caractérisé par**
un commutateur (1171) monté sur un chalumeau soudeur (1130) ou une gâchette de chalumeau qui active ledit microphone pour permettre audit dispositif de communication de recevoir des signaux depuis ledit microphone et dans lequel ladite image affiche des informations de réglage depuis ledit système d'alimentation en énergie de soudage.

2. Système de soudage selon la revendication 1, comprenant en outre un commutateur qui active ledit microphone pour permettre audit dispositif de communication de recevoir des signaux depuis ledit microphone.

3. Système de soudage selon la revendication 1 ou 2, dans lequel ledit mécanisme de génération d'informations génère une confirmation visuelle d'instructions audio reçues par ledit microphone.

4. Système de soudage selon l'une des revendications 1 à 3, dans lequel ledit casque (12) comprend en outre une batterie et un connecteur de charge (1191) à travers lequel passe une énergie électrique pour charger ladite batterie lorsque ledit connecteur de charge (1191) est couplé à une source d'énergie.

5. Système de soudage selon l'une des revendications 1 à 4, dans lequel ledit dispositif de communication est un dispositif de communication sans fil.

6. Système de soudage selon l'une des revendications 1 à 5, comprenant en outre un haut-parleur dans ledit casque qui est couplé audit dispositif de communication, dans lequel ledit haut-parleur fournit des signaux audio audit utilisateur.

7. Système de soudage selon la revendication 6, dans lequel lesdits signaux audio sont fournis depuis ladite alimentation en énergie de soudage.

8. Système de soudage selon l'une des revendications 1 à 7, dans lequel le système d'alimentation en énergie est configuré pour ne pas accepter et/ou ne pas permettre de changements par commande vocale de réglages de paramètre pendant que l'alimentation en énergie délivre un courant de soudage ou un courant de découpe plasma.

9. Système de soudage selon l'une des revendications 1 à 8, dans lequel le système d'alimentation en énergie est configuré pour permettre des changements de réglage pendant le soudage, dans lequel un signal audio/une indication/un mot spécifique est utilisé pour permettre une modification d'un paramètre, un tel signal audio/une telle indication/un tel mot spécifique étant distinct de sons de soudage normaux pour garantir d'éviter tout changement de paramètre par inadvertance.

10. Système de soudage selon l'une des revendications 1 à 9, dans lequel ledit mécanisme de génération d'informations génère une confirmation visuelle d'instructions audio reçues depuis ladite alimentation en énergie de soudage ; et/ou
dans lequel ledit casque comprend en outre un générateur de bruit pour interférer avec un bruit ambiant à l'intérieur dudit casque ; et/ou
dans lequel ladite alimentation en énergie comprend en outre un microphone et un haut-parleur pour permettre une communication audio depuis ladite alimentation en énergie de soudage audit casque.

11. Système de soudage selon l'une des revendications 1 à 10, dans lequel des réglages de sortie de ladite alimentation en énergie de soudage sont changés en utilisant lesdites instructions audio reçues par ledit microphone.

12. Système de soudage selon l'une des revendications 1 à 11, dans lequel ledit dispositif de communication se couple, avec une liaison de communication, à un deuxième dispositif de communication dans un deuxième casque de soudage de sorte qu'une communication audio entre ledit casque et ledit deuxième casque de soudage se déroule.

13. Système de soudage selon l'une des revendications 1 à 12, dans lequel lesdites informations de réglage sont réglées sur ladite alimentation en énergie de soudage sur la base desdites instructions audio.

14. Système de soudage selon l'une des revendications précédentes, comprenant :
un dispositif de sélection de paramètre de soudage pour sélectionner un paramètre de soudage contrôlable ;
dans lequel lesdites instructions audio incluent une première instruction qui augmente ledit paramètre de soudage contrôlable et une deuxième instruction qui réduit ledit paramètre de soudage contrôlable, et
dans lequel ladite alimentation en énergie de soudage augmente ledit paramètre de soudage contrôlable lorsque ladite première instruction est reçue et réduit ledit paramètre de soudage contrôlable lorsque ladite deuxième instruction est reçue.
